# EUROPEAN PATENT APPLICATION

(11) **EP 3 821 974 A1**
(43) Date of publication of application: **19.05.2021**
(21) Application number: 19209105.6
(22) Date of filing: 14.11.2019
(51) Int. Cl.: B01J 19/00, C07K 1/04

(54) **APPARATUS FOR ITERATIVE POLYMER SYNTHESIS**

(30) Priority: 13.11.2019 EP 19208979
(71) Applicant: Bachem AG, 4416 Bubendorf (CH)
(72) Inventor: Marchetti, Patrizia, 5000 Aarau (CH); Hübner, Sebastian, 79650 Schopfheim (DE); Eissler, Stefan, 4445 Häfelfingen (CH); Samson, Daniel, 4051 Basel (CH)
(74) Representative: Römer, Michael Werner

(57) **Abstract**

The present invention discloses an apparatus for fully automated iterative polymer synthesis at a large scale.

## Description

The present invention generally relates to the field of iterative polymer synthesis at an industrial scale. Improved apparatuses and methods for the fully automated synthesis of heteropolymers such as peptides and nucleic acids are disclosed.

Biopolymers such as proteins, peptides, nucleic acids and polysaccharides consist of a multitude of monomeric subunits, which are connected in a defined sequence to form a linear or branched polymer. This group of substances, in particular peptides, and nucleic acids, and man-made derivatives or variants thereof such as peptide nucleic acids and polynucleotide phosphorothioates, are of increasing interest as ingredients of pharmaceutical, cosmetic, and other industrial goods. Consequently, fully automated methods of synthesis at large scale are needed.

Chemical synthesis of biopolymers usually proceeds by iterative couplings of subunits to a growing molecule chain, which may be affixed to some type of support structure, e.g. to a particulate support or to a molecular tag determining solubility in specific solvents.

For example, in solid phase peptide synthesis (SPPS) a peptide anchored by its C-terminus to an insoluble polymer resin is assembled by the stepwise addition of the N-protected amino acid derivatives constituting its sequence. Successive cycles of amino acid addition are carried out, each usually comprising the steps of: a) cleavage of the N-protecting group from the resin-bound peptide, b) washing steps, c) coupling of a N-protected amino acid, and d) washing steps. Finally, the peptide is cleaved from the support and any remaining protecting groups, e.g. side-chain protecting groups, are removed. In the case of alpha amino acids, Nα-protected amino acid derivatives will be used, which may comprise additional side chain protecting groups. A similar cycle comprising coupling and deprotecting steps is involved in synthesis of nucleic acids, e.g. by the phosphoroamidite method of DNA synthesis.

The apparatus used for, e.g., solid phase peptide synthesis may comprise a first reactor, where the amino acid derivative is pre-activated, i.e. incubated in solution with coupling reagents to generate a reactive amino acid intermediate. The solution comprising the pre-activated amino acid may then be transferred to a second reactor comprising the SPPS resin, where the coupling reaction takes place. Alternatively, a solution comprising the amino acid may be added directly from a storage vessel to the SPPS resin inside the second reactor, and coupling reagents may be added *in situ.* If using sufficiently active amino acid derivatives such as active esters, the addition of coupling reagents might not be needed. The analogous observations apply to other types of iterative polymer synthesis.

Automated polymer synthesizers, e.g. for peptides and nucleic acids, exist and are commercially available. To our knowledge, these systems have in common that they rely on the dosing of liquid solutions of building blocks, e.g. of suitably protected amino acid derivatives, into the pre-activation reactor or into the second reactor, e.g. the SPPS reactor. One exemption is the setup of the ABI peptide synthesizer, where solid amino acid derivative samples are provided inside specific cartridges, which are loaded into the synthesizer. The synthesis then involves addition of solvent and activation reagents into the cartridge and transfer of the activated amino acid solution into the SPPS reactor. In other words: The amino acid cartridges correspond to a multitude of pre-activation reactors.

Both approaches are limited in their scalability: The provision of large amounts of amino acid solutions requires building of a tank farm, and the provision of a large number of subsequently used pre-activation reactors is not practical at an industrial scale. For this reason, SPPS at large scale is commonly performed in a semi-automated fashion where solutions of the appropriate amino acid derivatives are prepared manually by the operators.

There is a need for an apparatus allowing fully automated iterative polymer synthesis, in particular fully automated SPPS, at industrial scale, e.g. for the synthesis of 0.5 to 35 mol of polymer, preferably for the synthesis of 0.5 to 10 mol of polymer. Depending on the nature of the polymer, this may correspond to about 100g to 10 kg or 35 kg of product.

The present inventors have overcome this need by providing an apparatus suitable for fully automated iterative polymer synthesis, which is characterized in that the building block samples to be added may be stored in solid form and be added in solid form into a first reaction vessel RV1, where dissolution occurs. This approach involves the automated moving and dosing of solid particles/powders, which is much more demanding than the dosing of liquids with respect to the avoidance of clogging, non-quantitative transfer, and explosion hazards. In particular, the addition of powders into flammable solvents may be associated with the hazard of electrostatic ignition caused by friction during the transfer process itself. Moreover, carry-over of building block from one coupling cycle to the next should be avoided in order to avoid the formation of side-products. Surprisingly, the present inventors were able to overcome these challenges.

In general terms, the present invention may therefore relate to an apparatus suitable for performing iterative polymer synthesis, comprising:
a) a plurality of movable, closable storage vessels, which are shaped so as to enable transport by an automated means and each comprise a transfer port suitable for the transfer of solid material;
b) at least one first reaction vessel RV1, which comprises a transfer port suitable for the transfer of solid material, wherein:
   - said transfer port of the first reaction vessel RV1 is constructed and arranged such that it can be releasably docked to said transfer port of each storage vessel, thereby forming a connection between both vessels; and
   - material can pass though said transfer ports when in connected state, but not in disconnected state;
c) an automated transport means suitable for bringing a defined sequence of individual storage vessels to a specific first reaction vessel RV1 and away from it, wherein the automated transport means is capable of aligning said transfer port on the storage vessel with said transfer port on the first reaction vessel RV1 with sufficient precision to enable their docking to each other; and
d) at least one control unit CU1 controlling the actions of said automated transport means, the docking of said ports, and the opening and closing of the port.

Herein, the expression "iterative polymer synthesis" is used for processes of chemical synthesis, were a sequence of building blocks (B) is assembled into a polymeric chain by repeated cycles of building block addition. The building blocks will often represent monomeric units to be added to the growing polymer chain, but may likewise represent smaller fragments of the polymer to be synthesized such as dimers, trimers etc. For example, the building blocks used in peptide synthesis may commonly include derivatives of single amino acids as well as linear or branched dipeptide and tripeptide derivatives such as pseudoproline dipeptides. The building block addition may essentially represent a condensation reaction such as the formation of peptide bonds or of ester bonds, e.g. of phosphoester bonds. The cycles of building block addition may differ in detail, e.g. with respect to the conditions of building block coupling, but may share overall structure elements, e.g. the repetition of deprotection and coupling steps. The polymeric chain may take any conceivable shape, e.g. linear, circular, and/or branched. The single building blocks used may be the same or different. Preferably, a defined sequence of specific building blocks is used to build a specific polymer structure. This may be achieved by defining a sequence of individual storage vessels (each with a specific building block inside), which are to be used in subsequent cycles of building block addition in order to assemble the intended polymeric sequence. Typical examples of iterative polymer synthesis are -among many others-peptide synthesis (including synthesis of peptide derivatives and analogs) and polynucleotide synthesis (including synthesis of polynucleotide derivatives and analogs), e.g. by the phosphoroamidite method. In some embodiments , the iterative polymer synthesis is solid phase peptide synthesis (SPPS), in particular Fmoc-SPPS. In Fmoc-SPPS, a fluorenylmethoxycarbonyl protecting group (Fmoc) is used. The apparatus of the present invention is suitable for performing automated iterative polymer synthesis, in particular automated peptide synthesis such as automated solid phase peptide synthesis, e.g. automated Fmoc-SPPS.

Herein, the expression "polymer" is used for any molecular structure, which comprises a sequence of two or more monomeric units. Hence, the expression explicitly encompasses the commonly used expression "oligomer". Examples of polymers in the sense of the present application encompass dipeptides, tripeptides, other peptides, proteins, dinucleotides, trinucleotides, oligonucleotides, and polynucleotides, as well as any derivatives thereof.Preferably, the polymers to be synthesized comprise at least 3, at least 5, or at least 10 monomeric units. Preferably, the polymers to be synthesized comprise up to 30, up to 50, or up to 100 monomeric units. For example, the polymers to be synthesized may comprise 3 to 40, 5 to 40, 10 to 40, 3 to 50, 5 to 50, 10 to 50, 3 to 80, 5 to 80, 10 to 80, 3 to 100, 5 to 100, or 10 to 100 monomeric units. In the case of peptides, the monomeric unit may be delimited by two subsequent peptide bonds. The monomeric unit may be an amino acyl moiety or a derivative or an analogue thereof. The expression amino acyl moiety may refer to a moiety of the general formula I below, where: 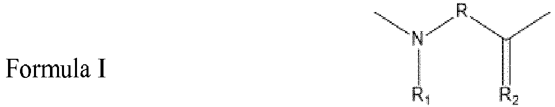
R1 is selected from the group consisting of H, alkyl, aryl, and arylalkyl;
R2 is selected from the group consisting of O and S; and
R is selected from the group consisting of:
   a linear or branched, substituted or unsubstituted alkylene,
   a linear or branched, substituted or unsubstituted heteroalkylene,
   a substituted or unsubstituted cycloalkylene,
   a linear or branched, substituted or unsubstituted arylalkylene,
   a linear or branched, substituted or unsubstituted heteroarylalkylene,
   a substituted or unsubstituted arylene,
   a substituted or unsubstituted heteroarylene.
Non-limiting examples for amino acyl moieties are the entities resulting from incorporation into the peptide chain of any of the amino acids occurring in natural peptides and proteins (including proline and hydroxyproline), of any substituted or unsubstituted α-ω amino acids in general, of p-amino benzoic acid, of 4-amino-cyclohexane-carbocylic acid, of piperidine-4-carboxylic acid, or of 8-amino-3,6-dioxa-octanoic acid (aka. AEEAc). As used herein, the expression "amino acid" relates to compounds comprising at least one deprotonated or protonated carboxyl group (-COO⁻ or -COOH) and at least one amino group, preferably a primary or secondary amino group (-NHR or -NH₂), which may be protonated. In the case of nucleic acids, the monomeric unit may be a nucleotide or a derivative or an analogue thereof.

The skilled person will immediately recognize that, according to the present invention, many different types of material transfer ports may be used. Preferably, the material transfer ports are amenable to cleaning in place, so as to avoid carry-over of building blocks between successive cycles of coupling. Preferably, the docking of the transfer ports results in an environmentally tight connection, i.e. it enables containment of the material transferred between the storage vessel and the reaction vessel, so as to avoid loss of reagents and contamination of the surroundings while charging the reaction vessel. Preferably, the material transfer ports -or at least the exposed surfaces thereof- are made of a material, which is essentially inert against the reagents it will be exposed to. Further, to minimize the risk of electrostatic ignition, it is preferred to use a conductive material. In some embodiments, the material transfer ports - or at least the exposed surfaces thereof-are made of stainless steel, Hastelloy alloys, or polymer-coated metal. Preferably, the material is compliant with the applicable regulations for the production of pharmaceutical products, cosmetics and/or food and beverages, i.e. it complies with good manufacturing practices (GMP). The skilled person will further understand that the inner diameter of the connected transfer ports' opening will influence the material transfer velocity and (s)he will chose this parameter in dependence of the flowability of the material to be transferred. For example, an inner diameter of about 10, 15, 20 or 30 cm may be suitable.

The expression "solid material" as used herein refers to chemical reagents, e.g. building blocks of the polymer to be synthesized, which are in solid state. The solid material may be a powder or be granular. The solid material may be in the form of pellets. The solid material may comprise macroscopic lumps. Preferably, the solid material may have the form of (amorphous or crystalline) powders or granules with a particle size in the range micrometers, e.g. in the range of 2 to 5000 micrometers, preferably 20 to 2000 micrometers. Some powders or granules may form macroscopic aggregates. Preferably, the powders or granules are flowable and/or easily soluble in the reaction solvent.

In some embodiments, the openings on the storage vessel and the first reaction vessel RV1 may each be closed by a ball valve, and the ports may be connectable, e.g., by a quick connect device, where the ball valves and the quick connect device are operated by a control unit. The control unit only allows for the opening of the ball valves after the docking of the ports, i.e. after the connection between both ports has been made. In order to facilitate, e.g., cleaning and filling of the vessels, the ball valves may be mounted on the respective vessel via a releasable connection such as a flange with screw threads or a clamp. In some embodiments, the ball valve on the first reaction vessel RV1 is mounted on a shock absorber such that vibrations resulting from the alignment and docking process are not transmitted to the first reaction vessel RV1.

In some embodiments, the material transfer ports on the storage vessel and the reaction vessel may each comprise one part of a split valve device. In such devices, each port may be closed by a flap. If both parts are positioned close to each other and aligned, they may be releasably docked together, whereby both flaps may be pressed together and become movable so as to open the valve and allow passage of material. The docking and undocking may be effected by an automatic drive integrated into the split valve device. In some embodiments, both flaps may be pressed together by applying vacuum suction to the space enclosed between them. The flap movement may be effected by an automatic drive integrated into one of the parts of the split valve device. The part of the split valve device comprising the drive(s) mediating docking/undocking and opening/closing of the valve is commonly referred to as the active part, the other as the passive part of the device. Preferably, the active part of the split valve device is mounted on the first reaction vessel RV1. As used herein, the expression "actions of the split valve device" may in particular comprise the docking/undocking of the parts of the split valve, the locking together of both parts, the unlocking of both parts, any integrated cleaning procedures for the flaps, and the opening/closing of the split valve device. At a minimum, the expression "actions of the split valve device" comprises the docking/undocking of the parts of the split valve and the opening/closing of the valve.

Examples of split valves comprise split butterfly valves and split cone valves. Such split valve devices are known in the art, e.g. from WO2010/092395, WO2010/145804 and EP1213244. Commercial sources include Müller, Sterivalves SRL, GEA Group, M.O. Industries, Visval and Chargepoint Technology Ltd, among many others. By their design (cf., e.g. Chimica Oggi (2003), 21(3/4), 78-79), split valve devices are suitable to contain the material transferred between the storage vessel and the reaction vessel, so as to avoid loss of reagents and contamination of the surroundings while charging the reaction vessel. Preferably, the parts of the split valve device are mounted on the respective vessel via a releasable connection such as a flange with screw threads or a clamp. This enables to remove the split valve, e.g. for cleaning purposes or in order to dispense the solid chemical reagents into the storage vessels. In some embodiments, the active part of the split valve device is mounted on a shock absorber such that vibrations resulting from the alignment and docking process are not transmitted to the first reaction vessel RV1. The precision of alignment needed for the active part and the passive part of the split valve device to dock depends on the specifications of the specific split valve device used. For example, the parts may need to be aligned with deviations from the ideal positioning not exceeding about 10-15 mm.

Thus, in some embodiments, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material;
b) at least one first reaction vessel RV1, which comprises the active part of said split valve device;
c) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other; and
d) at least one control unit CU1 controlling the actions of the automated transport means and of the split valve device.

The skilled person will understand that any automated transport means may be used, which is suitable for transporting the defined sequence of individual storage vessels to and from a specific first reaction vessel RV1 and to position them with sufficient precision to enable docking of the vessels' ports. In other words, the automated transfer means will align the ports of both vessels with the precision required by the port's docking mechanism.

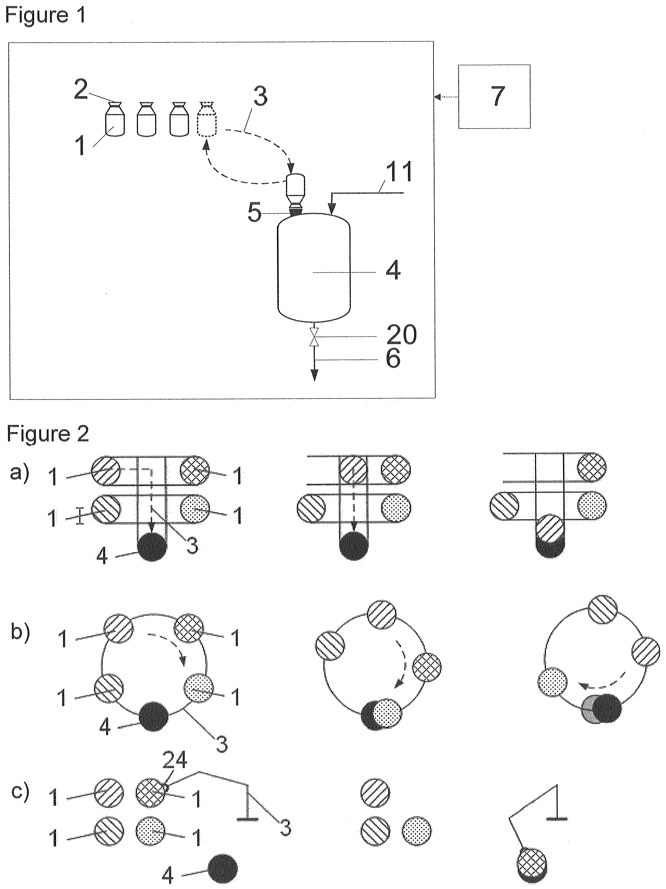

In some embodiments, the automated transport means comprises or essentially consists of a conveyor device. For example, a conveyor device may be used, which moves the storage vessels along a defined path, e.g. along rails installed above the first reaction vessel RV1 (cf., e.g., Figure 2a). As a further example, a conveyer wheel may be used, which brings a specific storage vessel into the docking position on top of the first reaction vessel RV1 by rotation around the wheel's central axis and downward movement of the storage vessel (cf., e.g., Figure 2b).

In some embodiments (cf., e.g., Figure 2c), the automated transport means comprises or essentially consists of a robot, e.g. a robotic arm. The robot or the robotic arm may be equipped with a gripping device. Such robotic arms are routinely used in industry and are readily available from commercial suppliers such as, e.g., Fanuc, Kuka, ABB, or Stäubli. Preferably, the robotic arm enables movement in three spatial dimensions and can rotate along each of the three axes of movement. The robotic arm may be fixed in place or may be movable along a guide. Preferably, the robot or robotic arm comprises a programmable logic controller (PLC), which may be part of the control unit CU1 and is controlled by a process control software installed on a remote supervisory computer.

It is preferable that the automated transport means, e.g. the robotic arm, is suitable for working in areas with risk of explosion, e.g. according to the ATEX specification of the European Union. The skilled person will routinely select a properly dimensioned automated transport means, which is suitable for lifting the weight of the filled storage vessel and which can transport the filled storage vessel to the transfer port of the first reaction vessel RV1 with suitable precision. For example, the automated transport means, e.g. the robotic arm, may be suitable for handling weights of 1kg to 500 kg, e.g. 10, 25, 50, 75, or 100 kg, and position the weight within 10 to 15 mm from their ideal position. Preferably, the automated transport means is further equipped with an optical device such as a camera or scanner to recognize a specific storage vessel by its machine-readable tag. The automated transport means may serve more than one first reaction vessel RV1. For example in this case, the automated transport means may comprise a programmable logic controller (PLC), which may be part of the control unit CU1 and is controlled by more than one process control software installed on one or more remote supervisory computer(s).

Gripping devices for transport devices, e.g. for robotic arms, are readily available. Preferably, the gripping device is a mechanical device, which grasps the storage vessel by direct impact on the vessel, e.g. on a protrusion of the vessel's walls or on a gripper plate mounted on the vessel. In some embodiments, a parallel gripper or a centric gripper with three or four fingers is used for this purpose. The gripping devices may be operated with pneumatic or electrical drives. In other embodiments, the gripping device may apply attractive forces such as vacuum suction, or a magnetic field to the storage vessel. In some embodiments, the gripping device allows a releasable grip and/or is suitable for lifting weights of 1kg to 500 kg, e.g. up to 10, 25, 50, 75, or 100 kg.

In some embodiments, the automated transport means essentially consists of a robotic arm, which is equipped with a releasable gripping device, such as a parallel gripper or a 3-finger centric gripper. For example the automated transport means may essentially consist of a robotic arm, which is movable in three spatial dimensions and can rotate along each of the three axes of movement, and which is equipped with a releasable gripping device such as a parallel gripper or a 3-finger centric gripper.

The skilled person is well aware of the fact that a number of different shapes may enable an automated transport means to take up and transport the storage vessel, and that the specific shape depends on the transport means chosen as well on the exact movements that need to be executed during the transport. In some embodiments, the body of the storage vessel may exhibit a (neck-like) narrowing such that an automated transport means may engage with the vessel. In some embodiments, the vessel may exhibit a protrusion or a hook, which an automated transport means may engage with. In some embodiments, the vessel may comprise a gripping plate, with which a gripping device can engage. The use of a gripping plate may be advantageous if the vessel needs to be turned upside down by the transport means and/or when additional elements such as a solvent line or a cleaning device need to be moved by the transport means.

In some embodiments, the automated transport means may bring the storage vessel with its material transfer port in docking position on top of the material transfer port of the reaction vessel, and the docking/undocking of both ports will be effected by drives integrated into the port. This may be the case, e.g. if using two parts of a split valve device as the material transfer ports. Alternatively, the automated transport means may bring the storage vessel in docking position and effect the docking/undocking. This may be the case, e.g., if using ball valves on each vessel and connecting the ports by a quick connect device.

As used herein, the expression "actions of the automated transport means" comprises in particular the selection of a specific storage vessel, the engagement with a storage vessel, the transport of the storage vessel to a specific first reaction vessel RV1 and away from it, and the alignment of the storage vessel in a specific position on the first reaction vessel RV1.

Herein, the expression "storage vessel" is used for a container, inside of which a material of interest can be stored under suitable conditions. The skilled person will define suitable conditions depending on the circumstances, e.g. so as to preserve the material's integrity or process safety according to the specifications of a given process.

In some embodiments, the storage vessel is shaped so as to enable transport by an automated means, and comprises a transfer port suitable for the transfer of solid material, wherein the material transport port is constructed and arranged such that:
- the transfer port may be releasably docked to a corresponding transfer port of a reaction vessel according to the invention, thereby forming an connection between both vessels; and
- material can pass though said transfer ports when in connected state, but not in disconnected state.
Preferably, said transfer port is the passive part of a split valve device, e.g. of a split butterfly valve.

The storage vessel may further comprise a liquid inlet and/or one or more machine-readable identification tags. In some embodiments, the present invention may relate to a movable, closable storage vessel for use in the apparatus according to the present invention, which is shaped so as to enable transport by an automated transport means and which comprises a machine-readable tag, a liquid inlet connectable to a mobile solvent line, and the passive part of a split valve device, which is suitable for the transfer of solid material. The movable, closable storage vessel for use in the apparatus according to the present invention, which may be equipped with a gripper plate, a machine-readable tag, a liquid inlet connectable to a mobile solvent line, and the passive part of a split valve device, which is suitable for the transfer of solid material. Preferably, the machine-readable tag is a bar-code, a QR code, or a vessel label in alphanumerical characters.

Preferably, the liquid inlet is positioned so as to enable purging the storage vessel with solvent. For example, the liquid inlet may be positioned opposite to the material transfer port, e.g. opposite to the passive part of the split valve device. In some embodiments, the storage vessel's liquid inlet feeds into a spray nozzle or spray ball inside the storage vessel. Preferably, the spray nozzle or ball is positioned in the top of the storage vessel, opposite to the passive part of the split valve device. The solvent line may be mobile and be transiently connected with the vessel's liquid inlet. Preferably, the vessel's liquid inlet comprises a part of a quick connect device, which corresponds to a part of a quick connect device mounted on the solvent line. Preferably, flow through the mobile solvent line is regulated by a device such as a valve and/or a pump under the control of the control unit CU1. The addition of solvent to the storage vessel may contribute to the completeness of material transfer from the storage vessel to the first reaction vessel RV1 by rinsing the internal surfaces of the storage vessel and/or by dissolving or suspending the solid material to be transferred.

Therefore, in some embodiments, the present invention relates to an apparatus for performing iterative polymer synthesis as described above, wherein each of the storage vessels comprises a liquid inlet, which allows connection to a (preferably mobile) solvent line, preferably via a quick connect device. In some embodiments the present invention relates to an apparatus for performing iterative polymer synthesis as described above, wherein each of the storage vessels comprises a machine-readable tag and a liquid inlet, which allows connection to a mobile solvent line, preferably via a quick connect device. In some embodiments, the present invention relates to an apparatus for performing iterative polymer synthesis as described above, wherein each of the storage vessels comprises a gripper plate, a machine-readable tag and a liquid inlet, which allows connection to a mobile solvent line. Preferably, a mobile solvent line may be reversibly connected to the storage vessel's liquid inlet. The connection may be established by the automated transport means. For example, the robotic arm detailed above may be capable of bringing a solvent line to the liquid inlet, keeping it in place, and removing it after the termination of solvent flow.

In some embodiments, the storage vessel may further be adapted to contain a protective gas. For example, the vessel may comprise a controllable valve, which can be connected to a vacuum source, and with a second controllable valve, which can be connected to a source of inert gas such as nitrogen. For automated inertization, the storage vessel may additionally comprise an electronic or mechanical pressure controller and the valves may be operated automatically. For manual inertization, the storage vessel may comprise a pressure indicator and manually controlled valves. The reaction vessel may further comprise sensors to monitor critical parameters such as, e.g., the temperature, the humidity, or the oxygen content inside the vessel.

The storage vessel(s) may be made of any suitable material, e.g. of metals, enamel, or polymers such as polypropylene, polyethylene, polyvinyl chloride, polystyrene, and poly ether ether ketone. Preferably, a material is chosen, which is essentially inert against the reagents it will be exposed to. Preferably, the material is compliant with the applicable regulations for the production of pharmaceutical products, cosmetics and/or food and beverages, i.e. it complies with good manufacturing practices (GMP). Further, to minimize the risk of electrostatic ignition, an electrically conductive material may be used. In some embodiments, the storage vessel is made of stainless steel or Hastelloy alloys or of a metal coated with an electrically conductive polymer. The size of the storage vessel can be chosen according to the scale of synthesis intended. In some embodiments, the storage vessel has an inner volume of about 1 to 200 liters, e.g. about 1, 5, 10, 15, 20, 25, 30, 35, 40, 45, 50, 100, 150, or 200 1. In some embodiments, the storage vessel has an inner volume of about 10 to 40 liters.

Herein, the expression "reaction vessel" or "reactor" is used for a container, which is suitable to take up the reagents of interest, and which has at least one liquid outlet. As such, the reaction vessel may be used to carry out a chemical reaction in the narrower sense of the term, i.e. a reaction, where covalent chemical bonds between atoms are made and/or broken. However, the reaction vessel may likewise be used to merely dissolve and/or mix the reagents of interest. The reaction vessel may be shaped so as to enable efficient mixing of its contents and rinsing of its walls. The reaction vessel will normally be closed, i.e. suitable to avoid unintended release of its contents. The reaction vessel may comprise additional inlets and outlets for materials, preferably for liquids. Such inlets will usually comprise a valve, which is closed unless material is purposefully being introduced into or let out of the reaction vessel. Preferably, material transfer through the additional inlets and outlets is driven and controlled by automatic devices (e.g. pumps and valves) governed by the control unit CU1.

As used herein, the designation RV1 is used to describe a first reaction vessel with an material transfer port, e.g. the active part of a split valve device, which may be coupled to a material transfer port, e.g. the passive part of a split valve device, on a storage vessel. Preferably, the first reaction vessel RV1 is a batch reactor. As will be detailed below, the designation RV2 is used herein to describe a second reaction vessel, which is connected to a first reaction vessel RV1 such that liquid can flow from the first reaction vessel RV1 into a second reaction vessel RV2. If no reference sign is used, the expression "a reaction vessel" as used herein may refer to a first reaction vessel RV1 and/or a second reaction vessel RV2.

The reaction vessel(s) RV1 and/or RV2 may preferably be adapted for working under protective atmosphere. For example, the reaction vessel(s) may comprise a first controllable valve, which can be connected to a vacuum source, a second controllable valve, which can be connected to a source of inert gas such as nitrogen, and an electronic or mechanical pressure controller. Preferably, the valves may be operated automatically and are under the control of the control unit CU1.

The reaction vessel/vessels may be made of any suitable material e.g. of metals, glass, enamel, or polymers such as polypropylene, polyethylene, polyvinyl chloride, polystyrene, and poly ether ether ketone. Preferably, a material is chosen, which is essentially inert against the reagents it will be exposed to. Further, to minimize the risk of electrostatic ignition, an electrically conductive material may be used. In some embodiments, the storage vessel is made of stainless steel or Hastelloy alloys or of a metal coated with an electrically conductive polymer. The size and dimensions of the reaction vessel can be chosen according to the scale of synthesis intended. For example, reactors are with an inner volume of 10, 30, 50, 75, 150, 200, 250, 300, 500, 750, or 1000 litres may be used. In some embodiments of the invention, the inner volume of the first reaction vessel(s) RV1 is about 30-250 litres, preferably about 40 to 200 litres, most preferably about 50 to 150 litres. As used herein, the expression "about" indicates that deviations by plus or minus 10% of the value given are possible.

The control unit CU1 controls at least the actions of the automated transport means, the docking and undocking of the material transfer ports connecting the storage vessel to the reaction vessel (e.g. of the parts of the split valve device), and the opening and closing of solid material transfer port (e.g. of the split valve device). The control unit CU1 may comprise several devices, which form different hierarchical levels of control, as is the case for a supervisory control and data acquisition (SCADA) control system architecture. For example, the control unit may comprise one or more remote supervisory computers, which gather data from and send control commands to peripheral devices, and one or more peripheral devices such as remote terminal units (RTU), programmable logic controllers (PLC) and user interfaces such as GUI panels. The PLCs and the supervisory SCADA software may receive input from field sensors such as sensors of temperature, pressure, level, weight, position, or concentration, amongst others. The one or more SCADA supervisory computing platform may additionally interact with a manufacturing execution system (MES), which in turn interacts with an enterprise resource planning (ERP) system. Moreover, the one or more SCADA supervisory computing platform may execute logging tasks by sending specific process parameters to a dedicated database. In some embodiments , the control unit comprises at least one SCADA system, at least one PLC with sensors and actors controlling the actions of the automated transport means, and at least one PLC with sensors and actors controlling the docking/undocking and opening/closing of the transfer port (e.g. the split valve device) and all other unit operations involving RV1 and/or RV2.

As will be detailed below, in some embodiments, the control unit CU1 may further control one or more elements selected from the list comprising: a means regulating the flow of the building block solution from one reaction vessel to another, the dosing of additional chemicals into the reaction vessel(s), the flow of protective gas (e.g. of nitrogen) into the reaction vessel(s), the mixing device(s) of the reaction vessel(s), the solvent flow into the reaction vessel(s) and into the storage vessel(s), the devices controlling the temperature and pressure inside the reaction vessel(s), the means for cleaning the active part on the split valve device located on the vessel(s), and the vibrator facilitating the solid material transfer into the reaction vessel(s).

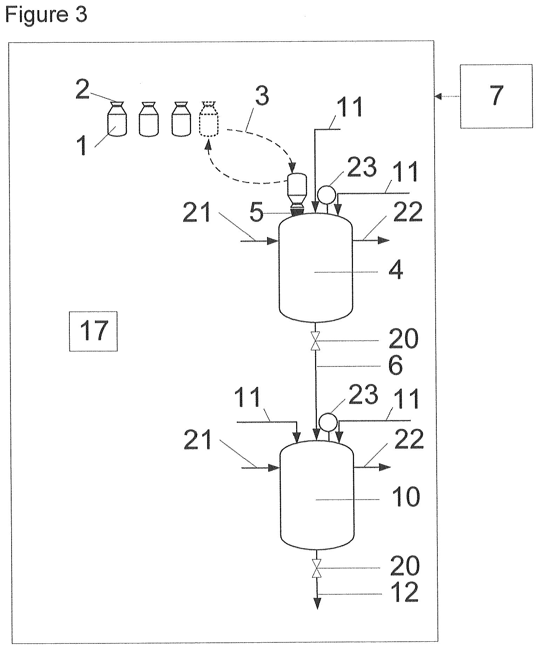

In some embodiments, the apparatus may be designed such that the dissolution of the building block, the optional activation of the building block, and its coupling to the growing polymer may occur inside the first reaction vessel RV1. In this case, the first reaction vessel RV 1 may be equipped with additional inlets for materials (e.g. activating reagents and additives), with a mixing device, and with a means for separating the growing polymer chain from the remaining components of the reaction mixture. In some embodiments, the apparatus may be designed such that the first reaction vessel RV1 may be used for dissolving and optionally pre-activating the building block, before transferring the building block solution to a second reaction vessel RV2, where the chemical coupling of the building block to the growing polymer will occur (cf., e.g., figure 3).

Therefore, in some embodiments, the apparatus may further comprise:
e) at least one second reaction vessel RV2 connected to at least one of the one or more reaction vessel(s) RV1
f) a device for effecting and/or controlling liquid flow from said first reaction vessel(s) RV1 into said connected second reaction vessel(s) RV2, which is controlled by the control unit CU1.

The first reaction vessel RV 1 may be equipped with additional inlets for materials (e.g. activating reagents and additives) and with a mixing device; and the second reaction vessel RV2 may be equipped with additional inlets for materials (e.g. activating reagents and additives), a mixing device, and a means for separating the growing polymer chain from the remaining components of the reaction mixture. The above specifications relating the first reaction vessel RV1 -in particular regarding inertization, the material and the volume of RV1- are likewise applicable to the second reaction vessel RV2. Commonly, the volume of the reaction vessels RV1 and RV2 will be similar within the same apparatus. Preferably, the second reaction vessel RV2 is a batch reactor. However, in some embodiments of the present invention, the second reaction vessel RV2 may be a solid phase reactor column.

Liquid flow between both the reaction vessels RV1 and RV2 may be effected by any means, e.g. by vacuum suction, by nitrogen pressure, by gravity, or by means of a pump. The flow may be controlled by regulating the respective means and/or by means of a valve.

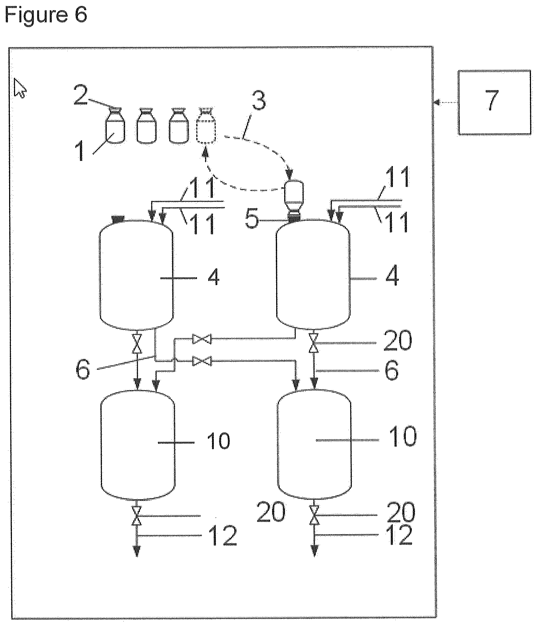

Depending on the synthesis pathway foreseen, it may be advantageous to integrate more than one first reaction vessel RV1 in the apparatus according to the present invention. For example, two first reaction vessels RV1 may be connected to a single second reaction vessel RV2. This then allows to separately prepare two building blocks to be used in the coupling reaction inside the second reaction vessel RV2. In the alternative, one first reaction vessel RV1 may be cleaned while preparing the building block to be used for the subsequent coupling step in the second reaction vessel RV2. Such a setup is particularly useful if the coupling step takes less time than the time of building block preparation. Additionally (cf. figure 6 for an example) or alternatively, one first reaction vessel RV1 may be connected to more than one second reaction vessel RV2. Such a setup is particularly useful if the same building block is used in parallel in both reaction vessels. Therefore, in some embodiments, the apparatus comprises a number of n first reaction vessels RV1 and a number of m second reaction vessels RV2, wherein n and m are integers chosen independently from the range of 1 to 10.

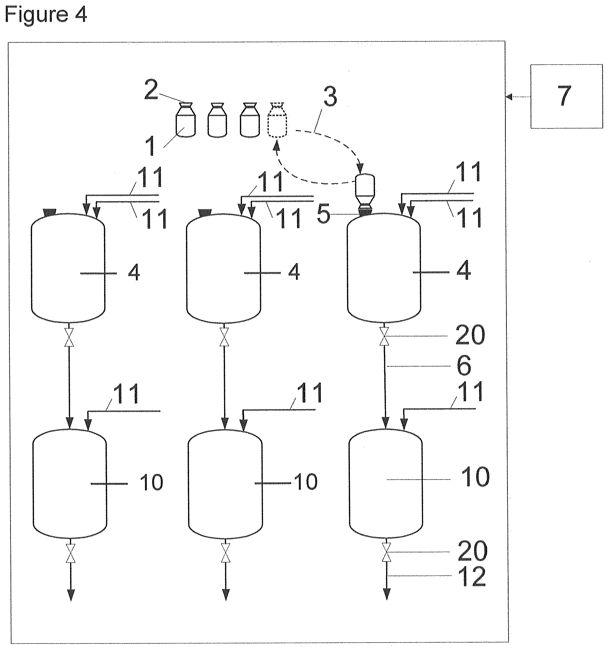

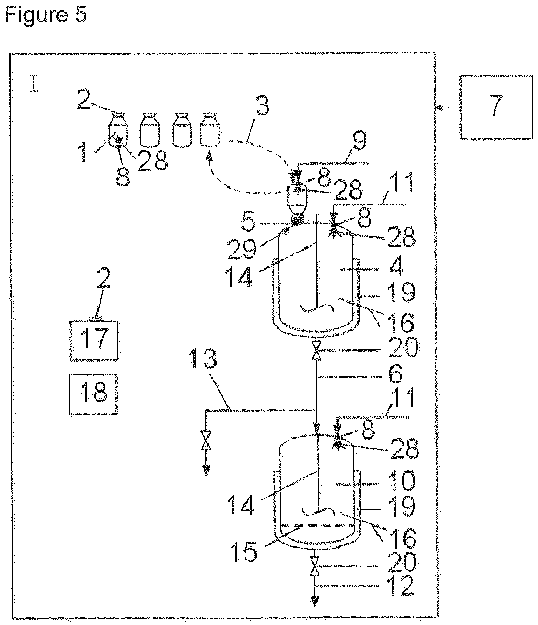

In some embodiments, the apparatus comprises one first reaction vessel RV1 and one second reaction vessel RV2 (cf. figure 5). In some embodiments, the apparatus comprises up to 6 -e.g. 2, 3, 4, 5, or 6- first reaction vessels RV1 plus up to 6 -e.g. 2, 3, 4, 5, or 6-second reaction vessels RV2, wherein each first reaction vessel RV1 is connected to exactly one second reaction vessel RV2 and a single automated transport means is used to load all first reaction vessels RV1 independently from each other with solid material (cf. figure 4 for an example). In some embodiments, the apparatus may comprise: up to 2, 3, 4, 5, or 6 first reaction vessels RV1; up to 2, 3, 4, 5 or 6 second reaction vessels RV2; or up to 6 first reaction vessels RV1 plus up to 6 second reaction vessels RV2.

In embodiments, where the apparatus comprises n first reaction vessels RV1ₙ and/or m second reaction vessels RV2ₘ, where n and m integers larger than 1 and equal to or smaller than 10, these reaction vessels and their accessory devices (such as solvent lines, sensors, temperature and pressure controls etc.) may be governed by the same control unit CU1, or by several independent control units CU1 to CUx, where x is an integer larger than 1. In the latter case, the independent control units may each have the architecture set out above with respect to CU1.

In order to control the amount and the concentration of building block within a first reaction vessel RV1 within narrow intervals, it is preferred to achieve a close to quantitative or quantitative transfer of the solid material from the storage vessel into the first reaction vessel RV1. Therefore, depending on the properties of the solid material to be transferred from the storage vessel into the reaction vessel, it may be advantageous to facilitate the material transfer between both vessels by means of a vibrator. The vibrator may be positioned permanently or transiently in the vicinity of the transfer ports or on the storage vessel. In one embodiment, the vibrator is permanently affixed to the active part of a split valve device on the first reaction vessel RV1. In one embodiment, to avoid shaking of the first reaction vessel RV1, the active part of the split valve device with the vibrator affixed to it may be mounted on a shock absorber. Preferably, the vibrator is suitable for working in areas with risk of explosion, e.g, according to the ATEX specification of the European Union. In some embodiments, the vibrator may be a pulsating vibrator. The vibrator may be selected from the group consisting of a pneumatic, electric, or hydraulic vibrator. Alternatively or in addition, in order to facilitate transfer of the solid material from the storage vessel to the first reaction vessel RV1, it may be advantageous to provide the storage vessel with a liquid inlet, as discussed above. This enables rinsing of the storage vessel with a suitable liquid, e.g. a solvent. Preferably, the control unit CU1 directs the action of the vibrator and the dosing of liquid into the storage vessel.

In some embodiments, the apparatus may further comprise a device M for monitoring the extent of material transfer from the storage vessel to the first reaction vessel RV1. As used herein, the expressions "device for monitoring the extent of material transfer", "device M for monitoring the extent of material transfer", and "device M" are synonymous and relate to a measuring device for a parameter, which changes as a function of material transfer from the storage vessel to the reaction vessel. The skilled person will understand that many different measuring devices may be used for this purpose. In some embodiments, the device M may be a scale, which is used to determine the weight or difference in weight of either the storage vessel or the reaction vessel after material transfer. In some embodiments, the device M may be an optical sensor, e.g. a UV/VIS probe, used to determine a concentration of the material transferred in the solution prepared within the first reaction vessel RV1. The amount of material transferred may then be inferred, e.g., either based on a known volume of solvent added, or on determination of the solution's volume using a level probe. Preferably, the control unit CU1 receives signals from the device M.

As explained before, the reaction vessel(s) RV1 and/or RV2 may contain a mixing device according to some embodiments. The skilled person will routinely choose the mixing device in dependence of the materials to be mixed, e.g. with respect of the physical robustness of the support used. For example, a stirrer comprising a rotating impeller may be used. Such an impeller may be a turbulent mixer causing axial, mixed, or radial flow of the liquid inside the reaction vessel. Known impellers include marine-type propellers, pitch-blade turbines, flat-blade turbines, and flat-blade paddles. The use of baffle blades may be helpful to improve mixing. Alternatively or in addition, mixing may be achieved by bubbling gas through the liquid. Alternatively or in addition, mixing may be achieved by liquid circulation, e.g. by means of a pumping circuit. A person skilled in the art will usually choose the mixing means so as to achieve efficient distribution of substances inside the reaction medium while avoiding foaming. When using solid supports inside the reaction vessel, the vessel and stirrer blades are preferably designed so as to minimize shearing forces. Preferably, the mixing device may be under the control of the control unit CU1.

In some embodiments, the reaction vessel(s) RV1 and/or RV2 will contain a means for separating the growing polymer chain from the reaction mixture within the reactor. The skilled person will immediately understand that the choice of this means will depend on the synthesis task at hand. For example, if performing solid phase synthesis on macroscopic particles (e.g. gel-like polymeric beads) as a solid support, these particles need to be separated from the surrounding liquid phase. For synthetic approaches, which rely on the use of a solubility modifying molecular tag, such as molecular hiving, a precipitate may need to be separated from a liquid. These tasks may be easily achieved by filtration. A frit or filter tissue integrated into the bottom part of the reactor may allow to drain the liquid from the reactor, while retaining the solid support or tag with the growing polymer chain inside. Other synthetic approaches may rely on the use of a solubility modifying molecular tag for partitioning between liquid phases. The separation of two liquid phases may be achieved by combining a controllable liquid outlet, which is positioned either at the bottom or at the side of the reactor, with a sensor for detecting the phase boundary, e.g. an optical sensor or a conductivity sensor. Preferably, the draining of liquid from the reactor is controlled by the control unit CU1, which may regulate the reactor's liquid outlet and -if working under inert gas- the pressure inside the reactor. Further, for synthetic approaches, which rely on molecular separation in liquid phase, a ultra- or nanofiltration membrane may be used to retain the growing polymer chain inside the reactor while washing out remaining components of the reaction mixture.

Hence, in some embodiments, the means for separating the growing polymer chain from the remaining components of the reaction mixture corresponds to a filter, e.g. to a fritted disk or to a single-use filter tissue. The filter may be made, e.g., of polymers such as polypropylene, stainless steel (or similar alloys), glass (sintered funnel like), or the like materials, and the pore size will be chosen depending on the size of the solid particles to be separated. For example, pore sizes in the range of 20 to 50 micrometer, e.g. about 25, 30, 35, 40, or 45 micrometer may be used. In some embodiments, the means for separating the growing polymer chain from the remaining components of the reaction mixture comprises a controllable discharge valve, a conductivity sensor, and a control element directing the state of the discharge valve, *inter alia* in dependence of the signal obtained from the conductivity sensor. As an alternative to conductivity sensors, optical sensors may be used. In some embodiments, the means for separating the growing polymer chain from the remaining components of the reaction mixture may correspond to a ultra- or nanofiltration membrane.

In order to determine and control specific reaction parameters, the reaction vessel(s) may be further equipped with one or more elements selected independently for each reaction vessel from the group consisting of a temperature sensor, a pressure sensor, a level sensor, a turbidity sensor, an optical sensor, a conductivity sensor, an impedance sensor, a heating device (e.g. a microwave system), a cooling device, a mixing device, a liquid port, a means for rinsing the inner walls of the reaction vessel(s), and a means for separating the growing polymer chain from the remaining components of the reaction mixture. The reaction vessel may be a jacketed reactor.

Preferably the apparatus disclosed herein is designed so as to avoid carry-over of reagents between successive coupling steps. This may involve the use of means for cleaning the active part of the split valve device on the first reaction vessel(s) RV1 and the use of a means for rinsing the inner walls of the reaction vessel(s) RV1 and/or RV2.

In principle, the cleaning of the active part of the split valve device may be achieved by connecting an empty storage vessel to the first reaction vessel RV1 to be cleaned and initiate solvent flow via the storage vessel's liquid inlet through the split valve device into the reaction vessel RV1. Preferably, the apparatus may comprise a cleaning device for the active part of the split valve device of the first reaction vessel(s) RV1, wherein the actions of the cleaning device are controlled by the control unit CU1. In some embodiments, said cleaning device comprises a passive part of the split valve device and at least one nozzle, which is connected to a solvent line. The cleaning device therefore can be connected to the active part of the split valve device so as to open the valve and clean its surfaces by solvent flow from the nozzle. Such cleaning devices are commercially available. They may be deployed to the first reaction vessel RV1 by the automated transport means, and their deployment and function may likewise be controlled by the control unit CU1. In some embodiments, the cleaning device and the storage vessels are each equipped with a gripping plate. In the alternative, commercially available split valve devices may comprise integrated cleaning devices, as is the case with, e.g., the solivalve® technology. The function of an integrated cleaning device may likewise be controlled by the control unit CU1.

Preferably, the reaction vessel RV1 and/or RV2 may comprise a liquid inlet, which allows connection to a solvent line. The liquid inlet may direct the solvent flow through a means for rinsing the inner walls of the reaction vessel. The means for rinsing the inner walls of the reaction vessel may be a spray ball or a nozzle, which is positioned in the top part of the reaction vessel and is connected to the liquid inlet. Rotating or fixed spray balls or nozzles may be used. Alternatively or in addition, the means for rinsing the inner walls of the reaction vessel may comprise liquid lines directing the flow against the vessel's walls. The liquid inlet may be permanently or releasably connected to a solvent line. The solvent line may be mobile and be transiently connected with the vessel's liquid inlet. Preferably, the vessel's liquid inlet comprises a flange, a clamp, or a part of a quick connect device, which corresponds to a flange, a clamp, or a part of a quick connect device mounted on the solvent line. Preferably, the solvent line further comprises a valve and/or a pump, which regulate(s) solvent flow through the solvent line and is operated by the control unit CU1. The means for rinsing the inner walls of the reaction vessel may be configured so as to rinse undissolved particles of the building block into the liquid contained at the bottom of the reaction vessel and/or to clean the reaction vessel of traces of the reaction mixture used. This may help to avoid carry-over of reagents from one coupling cycle to the next, thereby avoiding one source of by-product formation. The washing liquid drained from the reaction vessel may be monitored, e.g. by an optical sensor such as a flow cell or a UV/Vis probe, to control the rinsing or cleaning steps.

Depending on the setup of the apparatus the cleaning liquid may be collected at least partially inside the first reaction vessel RV1 in some embodiments. This option is of particular interest, if the coupling of the building block onto the growing polymer chain occurs inside the first reaction vessel RV1. In this case, the cleaning process, i.e. the rinsing of the first reaction vessel RV1's active part of the split valve device and the rinsing of the undissolved particles of the building block into the liquid contained at the bottom of the reaction vessel, may serve the double function of cleaning and of solvent addition into the first reaction vessel RV1. In this case, the solvent used for cleaning is also used for dissolving the building block and for carrying out the coupling reaction. After the coupling reaction is terminated, the rinsing of the first reaction vessel RV1's inner walls from any traces of the coupling reaction may serve the double function of rinsing the vessel and the solid support. Analogously, in some embodiments where the apparatus according to the present invention comprises a second reaction vessel RV2 connected to the first reaction vessel RV1, the cleaning liquid may be collected at least partially inside the second reaction vessel RV2. As an additional or alternative option, the apparatus may further comprise a waste line allowing to drain liquid from the first reaction vessel RV1 and from the line connecting the first reaction vessel RV1 with the second reaction vessel RV2 without passage of the liquid through RV2 (cf. figure 5 for an example). In this way, the cleaning of the first reaction vessel RV1 can proceed independently of the coupling reaction, which is carried out in the second reaction vessel RV2.

In some embodiments E1, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other; and
d) at least one control unit CU1 controlling the actions of the automated transport means, and of of the split valve device, the solvent flow into the liquid inlet of the storage vessel, the liquid flow into the liquid inlet of the first reaction vessel RV1, and the flow of inert gas through the first reaction vessel RV1.

In some embodiments E2, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) a cleaning device for the active part of the split valve device of the first reaction vessel(s) RV1;
c) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other; and
d) at least one control unit CU1 controlling the actions of the automated transport means, and of the split valve device the solvent flow into the liquid inlet of the storage vessel, the liquid flow into the liquid inlet of the first reaction vessel RV1, the cleaning device, and the flow of inert gas through the first reaction vessel RV1.

In some embodiments E3, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other;
d) a vibrator facilitating material transfer from the storage vessel to the first reaction vessel RV1; and
e) at least one control unit CU1 controlling the actions of the automated transport means, and of the split valve device, the solvent flow into the liquid inlet of the storage vessel, the liquid flow into the liquid inlet of the first reaction vessel RV1, the flow of inert gas through the first reaction vessel RV1, and the actions of the vibrator.

In some embodiments E4, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) a cleaning device for the active part of the split valve device of the first reaction vessel(s) RV1
d) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other;
e) a vibrator facilitating material transfer from the storage vessel to the first reaction vessel RV1; and
f) at least one control unit CU1 controlling the actions of the automated transport means, and of the split valve device the solvent flow into the liquid inlet of the storage vessel, the liquid flow into the liquid inlet of the first reaction vessel RV1, the flow of inert gas through the first reaction vessel RV1, and the actions of the cleaning device and the vibrator.

In some embodiments E5, the present invention relates to an apparatus suitable for performing iterative polymer synthesis, comprising:
a) a plurality of movable, closable storage vessels, which are shaped so as to enable transport by an automated means and each comprise the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) an automated transport means suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other;
d) a vibrator facilitating material transfer from the storage vessel to the first reaction vessel RV1;
e) a device M, preferably a scale, for monitoring the extent of material transfer between the storage vessel and the first reaction vessel RV1; and
f) at least one control unit CU1 receiving data from said device M and controlling the actions of the automated transport means and of the split valve device, the solvent flow into the liquid inlet of the storage vessel, the liquid flow into the liquid inlet of the first reaction vessel RV1, the flow of inert gas through the first reaction vessel RV1, and the actions of the vibrator.

In some embodiments E6, the present invention relates to an apparatus suitable for performing iterative polymer synthesis, comprising:
a) a plurality of movable, closable storage vessels, which are shaped so as to enable transport by an automated means and each comprise a gripper plate and the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) a cleaning device for the active part of the split valve device of the first reaction vessel(s) RV1;
c) an automated transport means comprising a gripping device capable of engaging with the storage vessels' gripper plate and suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other; and
d) at least one control unit CU1 controlling the actions of the automated transport means, of the gripping device, and of the split valve device, the solvent flow into the liquid inlet of the storage vessel, the liquid flow into the liquid inlet of the first reaction vessel RV1, the cleaning device, and the flow of inert gas through the first reaction vessel RV1.

In some embodiments E7, the present invention relates to an apparatus suitable for performing iterative polymer synthesis, comprising:
a) a plurality of movable, closable storage vessels, which each comprise a gripper plate the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) an automated transport means comprising a gripping device capable of engaging with the storage vessels' gripper plate and suitable for bringing a defined sequence of individual storage vessels to a specific first reaction vessel RV1 and away from it, wherein the automated transport means is capable of aligning said passive part of the split valve device with said active part of the split valve device with sufficient precision so as to enable their docking to each other;
d) a vibrator facilitating material transfer from the storage vessel to the first reaction vessel RV1 ;
e) a device M, preferably a scale, for monitoring the extent of material transfer between the storage vessel and the first reaction vessel RV1; and
f) at least one control unit CU1 receiving data from said device M and controlling the actions of the automated transport means, of the gripping device and of the split valve device, the solvent flow into the liquid inlet of the storage vessel, the liquid flow into the liquid inlet of the first reaction vessel RV1, the flow of inert gas through the first reaction vessel RV1, and the actions of the vibrator.

In some embodiments E8, the present invention relates to an apparatus suitable for performing iterative polymer synthesis, comprising:
a) a plurality of movable, closable storage vessels, which each comprise a gripper plate the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) a robotic arm equipped with a gripping device capable of engaging with the storage vessels' gripper plate, which robotic arm is suitable for bringing a defined sequence of individual storage vessels to a specific first reaction vessel RV1 and away from it, wherein the robotic arm is capable of aligning said passive part of the split valve device with said active part of the split valve device with sufficient precision so as to enable their docking to each other;
d) a vibrator facilitating material transfer from the storage vessel to the first reaction vessel RV1 ;
e) a device M, preferably a scale, for monitoring the extent of material transfer between the storage vessel and the first reaction vessel RV1; and
f) at least one control unit CU1 receiving data from said device M and controlling the actions of the robotic arm, of the gripping device, and of the split valve device, the solvent flow into the liquid inlet of the storage vessel, the liquid flow into the liquid inlet of the first reaction vessel RV1, the flow of inert gas through the first reaction vessel RV1, and the actions of the vibrator.

In some embodiments E9, the present invention relates to an apparatus suitable for performing iterative polymer synthesis, comprising:
a) a plurality of movable, closable storage vessels, which each comprise a gripper plate the passive part of a split valve device, which is suitable for the transfer of solid material, and a liquid inlet, which allows for connection to a solvent line, preferably wherein said inlet feeds into a means for rinsing the inner walls of the storage vessel;
b) at least one first reaction vessel RV1, which is adapted for working under protective atmosphere and comprises the active part of said split valve device, a liquid inlet feeding into a means for rinsing the inner walls of the first reaction vessel RV1, and a mixing device;
c) a cleaning device for the active part of the split valve device of the first reaction vessel(s) RV1, optionally wherein the cleaning device comprises a gripper plate;
d) a robotic arm equipped with a gripping device capable of engaging with the storage vessels' gripper plate, which robotic arm is suitable for bringing a defined sequence of individual storage vessels to a specific first reaction vessel RV1 and away from it, wherein the robotic arm is capable of aligning said passive part of the split valve device with said active part of the split valve device with sufficient precision so as to enable their docking to each other;
e) a vibrator facilitating material transfer from the storage vessel to the first reaction vessel RV1;
f) a device M, preferably a scale, for monitoring the extent of material transfer between the storage vessel and the first reaction vessel RV1; and
g) at least one control unit CU1 receiving data from said device M and controlling the actions of the robotic arm, of the gripping device, of the split valve device, and of the cleaning device, the solvent flow into the liquid inlet of the storage vessel, the liquid flow into the liquid inlet of the first reaction vessel RV1, the flow of inert gas through the first reaction vessel RV1, and the actions of the vibrator.

In another embodiment E10, the apparatus according to the above embodiments E1 through E9 may further comprise:
- at least one second reaction vessel RV2, which is connected to at least one of the one or more first reaction vessel(s) RV1, is adapted for working under protective atmosphere, and comprises a mixing device and a liquid inlet, preferably wherein said inlet is connected to a solvent line and feeds into a means for rinsing the inner walls of the second reaction vessel RV2;
- a controllable means for effecting and/or controlling liquid flow from said first reaction vessel(s) RV1 into said connected second reaction vessel(s) RV2; wherein
- the control unit CU1 further controls the liquid flow into the liquid inlet of the second reaction vessel RV2, the flow of inert gas through the second reaction vessel RV2, and the means for effecting liquid flow from said first reaction vessel(s) RV1 to said second reaction vessel(s) RV2.
- In another embodiment E11, the apparatus according to the above embodiments E1 through E9 may further comprise: at least one second reaction vessel RV2, which is connected to at least one of the one or more first reaction vessel(s) RV1, is adapted for working under protective atmosphere, and comprises a mixing device and a liquid inlet feeding into a means for rinsing the inner walls of the second reaction vessel RV2;
- a controllable means for effecting and/or controlling liquid flow from said first reaction vessel(s) RV1 into said connected second reaction vessel(s) RV2;
- a waste line allowing to drain liquid from the first reaction vessel RV1 and from the line connecting the first reaction vessel RV1 with the second reaction vessel RV2 without passage of the liquid through the second reaction vessel RV2; wherein
- the at least one control unit CU1 further controls the solvent flow into the liquid inlet of the second reaction vessel RV2, the flow of inert gas through the second reaction vessel RV2, the cleaning device for the active part of the split valve device, and the means for effecting and/or controlling liquid flow from said first reaction vessel(s) RV1 to said second reaction vessel(s) RV2.

In each of the above embodiments E1 through E11, the reaction vessel(s) RV1 and/or RV2 may further comprise a means for separating the carrier with the growing polymer chain from the remaining components of the reaction mixture. In particular, the reaction vessel(s) RV1 and/or RV2 may comprise a frit or filter tissue integrated into the bottom part of the reactor.

The present invention further relates to a method of iterative polymer synthesis comprising the use of an apparatus or of a storage vessel according to the present invention.

The explanations and definitions set out above with respect to the apparatus of the present invention are likewise applicable with respect to the methods of the present invention, and vice versa. Preferably, the method of iterative polymer synthesis is carried out in automated fashion.

As explained before, the apparatuses of the present invention may be particularly advantageous in that they may allow to perform the steps of iterative polymer synthesis in automated fashion. As used herein, the expression "in automated fashion" describes a process, which is routinely executed without the intervention and without the permanent control by a human being. This may mean that the steps of the synthesis process are governed by a control unit, e.g. that the steps of the synthesis are under the control of the control unit CU1 detailed above. However, the apparatus and/or control unit may be configured so as to allow for or even request the intervention by a human being under specific circumstances. This may occur, e.g., in case of unforeseen events, such as process parameters being outside of specific predefined ranges. Moreover, it may be advantageous to be able and carry out, e.g., a certain number of coupling cycles in automated fashion, while executing a specific critical step under human control and/or with human intervention.

The steps of the methods detailed herein may be executed in the exact order, in which they are recited below. However, the skilled person is aware of the fact that the order of steps may be different in some cases. As used herein, the expression "the following steps 1 to x are carried out" refers to a process, where each of the steps 1 to x are carried out, although not necessarily in the order indicated. For example, it will be immediately clear to the skilled practitioner that the below steps 1) to 10) and i) to x) do not need to be carried out in the order indicated. Moreover, one or more single step(s) may be repeated several times throughout the sequence of steps. For example, steps v) and vi) may occur simultaneously, and/or step 9) may occur several times before and during step 10).

In some embodiments, the present invention relates to a method of iterative polymer synthesis, wherein the following steps 1) to 10) are carried out at least once in automated fashion:
1) Providing a plurality of movable, closable storage vessels, which are shaped so as to enable transport by an automated means and each comprises a passive part of a split valve device suitable for the transfer of solid material, wherein each of the storage vessels contains a defined amount of a building block B in solid form to be used in one cycle of the iterative polymer synthesis process;
2) Transferring a specific storage vessel selected from said plurality of storage vessels to a first reaction vessel RV1, which comprises an active part of said split valve device using an automated transport means;
3) Aligning and docking the passive part of the split valve device on said storage vessel to the active part of the split valve device on said first reaction vessel RV1;
4) Opening the split valve device and transferring the building block B from said storage vessel into said first reaction vessel RV1;
5) Disolving said building block B by addition of a suitable solvent, thereby forming a solution of the building block B inside the first reaction vessel RV1;
   - Optionally, addition of one or more activating reagents to said building block B inside the first reaction vessel RV1
6) Transfer of the solution obtained in step 5), to a second reaction vessel RV2 containing a carrier, to which a molecule C is tethered, thereby obtaining a reaction mixture containing the building block B and the carrier with the molecule C;
7) Cleaning the first reaction vessel RV1, including the active part of the split valve device, by rinsing with solvent, which may be the same as or different from the solvent used in step 5);
8) Incubating of the reaction mixture obtained in step 6) under conditions, which allow for the formation of a chemical bond between the building block B and the molecule C so as to form a molecule C', which is extended by one building block unit;
   - Optionally, addition of one or more activating reagents to the contents of said the second reaction vessel RV2
9) Retaining the carrier with the extended molecule C' inside the second reaction vessel RV2 (10), while purging a liquid comprising residual educts and byproducts of the coupling reaction from the second reaction vessel RV2;
10) Conditioning the carrier with the extended molecule C' for the next coupling round with the extended molecule C' as molecule C.

In some embodiments, the present invention relates to a method of iterative polymer synthesis, wherein the following steps i) to x) are carried out at least once in automated fashion:
i) Providing a plurality of movable, closable storage vessels , which are shaped so as to enable transport by an automated means and each comprise a passive part of a split valve device suitable for the transfer of solid material, wherein each of the storage vessels contains a defined amount of a building block B in solid form to be used in one cycle of the iterative polymer synthesis process;
ii) Providing a first reaction vessel RV1, which comprises an active part of said split valve device, and which contains a carrier, to which a molecule C is tethered;
iii) Transferring a specific storage vessel selected from said plurality of storage vessels to said first reaction vessel RV1 containing the carrier with the molecule C using an automated transport means;
iv) Aligning and docking of the passive part of the split valve device on said storage vessel to the active part of the split valve device on said first reaction vessel RV1;
v) Opening of the split valve device and transferring the amount of the building block B from said storage vessel into said first reaction vessel RV1;
vi) Dissolving said building block B by addition of a suitable solvent, thereby forming a reaction mixture containing the building block B and the carrier with the molecule C inside the first reaction vessel RV1;
   - Optionally, addition of one or more activating reagents to the contents of said first reaction vessel RV1
vii) Incubation of the reaction mixture obtained in step vi) under conditions, which allow for the formation of a chemical bond between the building block B and the molecule C so as to form a molecule C', which is extended by one building block unit;
   - Optionally, addition of one or more activating reagents to the contents of said first reaction vessel RV1
viii) Retaining the carrier with the extended molecule C' inside the first reaction vessel RV1, while purging a liquid comprising byproducts and residual educts of the coupling reaction from the first reaction vessel RV1
ix) Rinsing the first reaction vessel RV1, including the active partof the split valve device, with a solvent, which may be the same as or different from the solvent used in step vi);
x) Conditioning the carrier with the extended molecule C' for the next coupling round with the extended molecule C' as molecule C.

In some embodiments, the above steps 1) to 10) or i) to x) are carried out in automated fashion in at least two subsequent cycles of building block addition. In some embodiments, the above steps 1) to 7) or i) to vii) are carried out in automated fashion for the majority of cycles of building block addition, preferably for each cycle of building block addition.

As has been explained above, the expression "building block" as used herein relates to a unit to be added to the polymer chain. The reference sign building block "B" is added to facilitate reading. The building block may include protecting groups, which block unintended side reactions of some of its functional groups. The building block may be capable of reacting with the molecule C without the addition of further activating reagents, as is the case for active esters. In the case of peptide synthesis, the building block B may be an amino acid derivative. Said derivatives may comprise N-protecting groups, such as a tert. butyloxycarbonyl (Boc), a 9-fluorenylmethyloxycarbonl (Fmoc), or an allyloxycarbonyl (Alloc) amino protecting group and may further exhibit suitable side chain protecting groups. In a some embodiments, the building block B may be an amino acid derivative, preferably a N-protected amino acid derivative. Suitable Fmoc amino acid derivatives for use with some embodiments of the present invention comprise, e.g., the standard compound Fmoc-Ala-OH, Fmoc-Arg(Pbf)-OH, Fmoc-Arg(Pmc)-OH, Fmoc-Asn(Trt)-OH, Fmoc-Asn(Mtt)-OH, Fmoc-Asp(OtBu)-OH, Fmoc-Asp(OMpe)-OH, Fmoc-Cys(Trt)-OH, Fmoc-Cys(Mmt)-OH, Fmoc-Gly-OH, Fmoc-Gln(Mtt)-OH, Fmoc-Gln(Trt)-OH, Fmoc-Glu(OtBu)-OH, Fmoc-His(1-Trt)-OH, Fmoc-Ile-OH, Fmoc-Leu-OH, Fmoc-Lys(Boc)-OH, Fmoc-Met-OH, Fmoc-Phe-OH, Fmoc-Pro-OH, Fmoc-Ser(tBu)-OH, Fmoc-Thr(tBu)-OH, Fmoc-Trp(Boc)-OH, Fmoc-Tyr(tBu)-OH, and Fmoc-Val-OH, which are commercially available from various sources. It should be noted that the use of non-natural amino acid derivatives such as Aib (α-aminoisobutyric acid), Nle (norleucine), Orn (ornithine), or derivatives of substituted or unsubstituted a-w amino acids in general is likewise encompassed by some embodiments of the methods of the present invention. Depending on the synthesis strategy chosen, peptide derivatives such as pseudoproline dipeptide derivatives, di-or tripeptide derivatives, or branched dipeptide derivatives may be used in lieu of single amino acid derivatives. As used herein, the expression "amino acid derivative" encompasses all of said building blocks.

According to the present invention, at least a part of the building block B may be transferred in solid form, i.e. as a powder or granules, from the storage vessel into the first reaction vessel RV1. As has been detailed above, this may be effected via a suitable port, e.g. via a split valve device, thus ensuring that no material is lost to and contaminates the outside. The material transfer may be aided by mechanical means such as a vibrator applied to the port or the storage vessel and may be improved by subsequent rinsing of the storage vessel with solvent sprayed into the storage vessel from a suitable liquid inlet. It may be advantageous to control the extent/completeness of the material transfer via a measuring device such as a scale or an optical probe. In the case of peptide synthesis, the great majority of building blocks used will be in solid state at ambient temperature. It should however be noted that the present application can likewise be used with a liquid building block. In this case, one or more storage vessel(s) comprising said liquid building block is/are added to the above-mentioned plurality of storage vessels containing building blocks in solid state.

The solvent used for dissolving the building block B may be compatible with the coupling reaction. Preferably, the solvent does not react itself with either the building block, the molecule C or C', or any other reagents present in the coupling reaction. Preferably, the building block B is easily soluble in the solvent used. For example, the building block B may be soluble in the solvent at room temperature at a concentration of at least 50 mg/ml, at least 100 mg/ml or at least 200 mg/ml. In some embodiments, the solvent used is selected from the group consisting of N-methylpyrrolidone (NMP), dimethylformamide (DMF), N-butyl-pyrrolidone (NBP), dimethyl isosorbide (DMI), gamma-valerolactone (GVL), dihydrolevo-glucosenone (Cyrene), dimethyl sulfoxide (DMSO), tetra-hydropyran (THP), tetrahydrofuran (THF), 2-methyltetrahydrofuran (Me-THF), 1,3-dioxolane, ethyl acetate (EtOAc), dichlormethane, acetonitrile, and toluol. The solvent may be dosed into the storage vessel and/or into the first reaction vessel RV1 and/or into the second reaction vessel RV2 via appropriate inlets located on either the reaction vessel or a storage vessel or cleaning device connected to it. Dissolution of the building block may be facilitated by a mixing means.

As used herein, the term "carrier" refers to any macroscopic or molecular structure, to which the molecule C can be tethered and which enables separation of the molecule C from a liquid phase. Hence, the term encompasses the terms "resin" and "[resin]", which may be understood in the broadest sense as a particulate structure usable for solid phase synthesis, in particular for solid phase peptide synthesis (SPPS). The terms "resin", "solid support" and "solid phase" are used exchangeably herein. Further, the term "carrier" encompasses any molecular tags allowing for separation of the tag with the molecule C tethered to it from a liquid phase. Typical examples for this latter type of carrier are the proprietary hydrophobic tags of the Molecular Hiving™ system. Based on the foregoing, it will be clear to the skilled artisan that the term "carrier" as used herein refers to a population of entities, e.g. of bead-like particles or of molecular tags, and is used in the singular form only for the sake of readability.

As used herein, the expression "molecule C" relates to the growing polymer chain, which is tethered to the carrier. Typically, the molecule C corresponds to the first building block of the polymeric chain at the beginning of the synthesis and grows in each cycle of synthesis by one building block unit, e.g. by one monomeric unit. The molecule C may comprise additional (side chain) protecting groups, which may be removed at the end of the synthesis. While the "molecule C" is referred to herein in the singular form for the sake of readability, the skilled person will understand that the expression relates to a population of synthesis products, most of which exhibit the same chemical structure. The molecule C may be tethered in any way to the carrier. It may be bound via a covalent chemical bond or via non-covalent bonds mediated by, e.g. ionic interactions, van der Waals bonds, or hydrogen bridges. The molecule C may be tethered to the carrier via an affinity tag. In SPPS, a common way of tethering the growing peptide chain to the carrier (aka. resin) is via a chemical bond between the C-terminal carboxyl group and the carrier. As an alternative, the peptide may also be conjugated to the resin via a side chain of a (preferably terminal) amino acid.

SPPS is commonly carried out on gel phase rather than solid phase supports. Suitable resins may be based on polystyrene, polystyrene-PEG composites, PEG, PEGA, crosslinked ethoxylate acrylate (CLEAR), polyamides, polydimethylacrylamide, or any other support with the desired physical and chemical properties. Resins based on beaded polystyrene with 1% divinylbenzene are among the routinely used supports, typically having a size distribution of 200-400 mesh or 100-200 mesh. Polystyrene based 4-Alkoxybenzyl alcohol (Wang) resin, diphenyldiazomethane (PDDM) resin, 4-(2',4'-Dimethoxyphenyl-Fmoc-aminomethyl)-phenoxymethyl-polystyrene (Rink) resin, 2-Methoxy-4-alkoxybenzyl alcohol (Sasrin) resin, 2-Chlorotrityl chloride (CTC), and CTC-amidomethyl resin are particularly suitable and are commercially available from suppliers such as Sigma-Aldrich, Bachem and EMD Millipore. However, any other resin suitable for SPPS may be used. The resins are typically swollen in a suitable solvent for use in SPPS. In some embodiments, in step 7) or step ii) of the methods according to the present invention, the carrier with the molecule C may be provided as a drained cake or as a slurry of swollen beads inside the reaction vessel; or it may be provided as a solution of the carrier with the molecule C in a suitable solvent.

In some embodiments, the method involves the addition of activating reagents and/or additives facilitating the coupling reaction to the first reaction vessel RV1 and/or to the reaction vessel RV2. For example, the activating reagents and/or additives may be added after step 5) and/or during step 8) of the above method. As a further example, the activating reagents and/or additives may be added after step vi) and/or during step vii) of the above method. In the case of peptide synthesis, common activating reagents and additives comprise, among many others: N,N'-diisopropylcarbodiimide (DIC) with either OxymaPure® or hydroxybenzotriazole (HOBt); (benzotriazolyl) tetramethyluronium tetrafluoroborate (TBTU) or DEPBT plus one or more bases, preferably N,N-diisopropylethanolamine (DIPEA); (7-Azabenzotriazolyl) tetramethyl uronium tetrafluoroborate (TATU) plus DIPEA; (Benzotriazole-1-yl) tetramethyluronium hexafluorophosphate (HBTU) plus DIPEA; and O-(7-Azabenzotriazolyl)-tetramethyluronium hexafluorophosphate (HATU) plus DIPEA. The reagents may be dosed into the reaction vessel RV1 and/or RV2 in liquid form, preferably via an automated pump. The skilled practitioner is aware of the fact that a wide range of incubation times (e.g. from minutes to hours) and temperatures (e.g. from 15 to 90 °C) may be suitable for allowing the coupling reaction to occur. (S)he will optimize and select the specific coupling conditions dependent on the task at hand. It will be immediately clear to the skilled practitioner that, depending on the synthesis strategy chosen, the coupling reaction may occur inside a suspension, an emulsion, or a homogenous solution.

In step 7), the cleaning of the first reaction vessel RV1 may be carried out. Preferably, this step comprises extensive rinsing with solvent and removal of traces of the building block from the transfer port, as has been detailed above with respect to the apparatus for performing iterative polymer synthesis. The cleaning of the first reaction vessel RV1 may be carried out in parallel with step 8). In some embodiments, where the chain elongation reactions occurs inside the first reaction vessel RV1, step ix) -i.e. the rinsing of the first reaction vessel RV1 including the active part of the split valve device with a solvent- may likewise involve removal of traces of the building block from the transfer port, as has been detailed above with respect to the apparatus for performing iterative polymer synthesis. In such embodiments, the washing liquid will also contact the carrier with the extended molecule C' and/or with the not (yet) extended molecule C.

The step 10) or x) of the method -i.e. the step of conditioning the carrier with the extended molecule C'- may comprise the rinsing of the carrier with the extended molecule C' so as to remove or reduce the concentration of any reagents used in the previous round of building block addition. Optionally, the step 10) or x) of the method may involve the capping of unreacted molecules C, e.g. by acylation. The addition of capping reagent to the reaction vessel comprising the molecule C and the subsequent removal of these reagents from the reaction vessel may be effected in automated fashion by valves and pumps under the control of the control unit CU1. Commonly, the step of conditioning the carrier with the extended molecule C' may further involve the removal of a protecting group from the molecule C in order to prepare for the next round of building block addition. For example, in the case of Fmoc SPPS, the Fmoc group may be cleaved by a treatment with base. Popular bases for this purpose comprise, e.g., secondary amines such as piperidine and 4-methyl piperidine. Suitable solvents comprise, e.g., DMF, NMP, dimethyl sulfoxide, dichlormethane, tetrahydrofuran, acetonitrile, toluol, and mixtures thereof. The skilled practitioner is aware of the fact that a wide range of incubation times (e.g. from minutes to hours) and temperatures (e.g. from 15 to 90 °C) may be used.

The following Figures and Examples, including the experiments conducted and the results achieved, are provided for illustrative purposes only and are not to be construed as limiting the scope of the claims. It should be noted that the embodiments described throughout this document may be generally combined with each other.

### Examples

### Example 1: Flowability and Solubility of amino acid derivatives

The flowability of 15 samples of amino acid powders was tested. The flowability of each powder in unconfined state was assessed by powder flow analysis in a rotating drum ("revolution analyzer"), by testing mass flow through a funnel, and by determining the angle of repose. The latter two measurements were performed according to chapter 1174 of the U.S. Pharmacopeia. The flowability of the samples at a confided state was analyzed by measurements with an annular shear cell and by using the Evolution Powder Tester device by PS Prozesstechnik GmbH, Basel. In these tests, the samples were initially densified with different pre-consolidation forces.

In the tests without confinement, Fmoc-Cys(Trt)-OH and Fmoc-Met-OH consistently stood out for good flowability, and flowability parameters for Fmoc-Gly-OH were likewise good. On the other hand, poor flowability parameters were determined for Fmoc-Ala-OH and Fmoc-Pro-OH. Interestingly, in the tests with confinement, Fmoc-Cys(Trt)-OH and Fmoc-Pro-OH consistently stood out for good flowability. Medium values were determined for Fmoc-Ala-OH, while Fmoc-Gly-OH and Fmoc-Lys(Boc)-OH consistently stood out for poor flowability. This might indicate that Fmoc-Gly-OH may lose its flowability upon compression. On the other hand, the compression may have broken down lumps in the Fmoc-Pro-OH sample, thereby increasing its flowability.

The concentration of the same amino acids in saturated solutions of dimethyl formamide at room temperature was determined by analytical reversed phase ultrahigh pressure liquid chromatography. The values ranged from below 200 mg/ml for Fmoc-Pro-OH over around 400 mg/ml for Fmoc-Ala-OH to above 600 mg/ml for Fmoc-Gly-OH.

### Example 2: Transfer of an amino acid derivative powder through a split valve device

Fmoc-Ala-OH and Fmoc-Gly-OH were chosen as test substances because of their poor flowability revealed by the previous experiments.

2 kg of amino acid derivative powder were filled into a 25 1 storage vessel made of stainless steel. The storage vessel was closed by the passive part of a DN100 split valve device, i.e. a split valve device with an inner opening of roughly 10 cm. The active part of the split valve device was docked and a transparent bag attached to it. The split valve device was opened manually and the flow of the powder observed by eye though the transparent bag. A pulsating vibrator was applied to the active part of the split valve device to facilitate the powder flow. The powder transfer was completed within a maximum time of 5 minutes. Visual inspection was performed by opening the valve. Remnants of white powder were observed on the walls of the vessel and valve an on the valve's flap.

The vessel was subsequently rinsed with ca. twenty liters of dimethyl formamide though a fixed spray ball, which was placed inside the storage vessel opposite to the split valve device and was connected to a solvent line. Visual inspection was repeated after rinsing. No remnants were observable.

These findings demonstrate that the powder transfer is achievable within a suitable time frame and that efficient rinsing of the split valve device is possible. They therefore can be considered a proof of concept for the present invention.

### List of reference signs

1 storage vessel
2 passive part of split valve device
3 automated transport means
4 first reaction vessel RV1
5 active part of split valve device
6 liquid connection line
7 control unit CU1
8 liquid inlet
9 mobile solvent line
10 second reaction vessel RV2
11 liquid line
12 drain
13 waste line
14 mixing device
15 separation means
16 sensor
17 cleaning device
18 device M for monitoring material transfer
19 heating/cooling device
20 device controlling liquid flow
21 connection to source of protective gas
22 connection to vacuum source
23 pressure controller
24 gripping device
25 gripper plate
26 first solid material transfer port
27 second solid material transfer port
28 rinsing means
29 liquid port

### Brief Description of the Figures

**Figure 1** shows a basic layout of some embodiments according to the present invention. Out of a plurality of such storage vessels, an automated transport means (3) selects a storage vessel (1) comprising a passive part (2) of a split valve device. The automated transport means (3) transfers said storage vessel (1) to a first reaction vessel RV1 (4), and aligns the passive part (2) of the split valve device with an active part (5) of said split valve device on the first reaction vessel RV1. The active and passive parts (2, 5) of the split valve device are docked and the valve opened to allow for passage of solid material from the storage vessel (1) into the first reaction vessel RV1 (4). After the transfer, the split valve device is closed and undocked and the storage vessel (1) is transported away from the first reaction vessel RV1 (4). Liquid may be added to the first reaction vessel RV1 (4) via a liquid line (11) and drained via a line (6). A device controlling liquid flow (20) is integrated into the liquid line (6). The device for controlling liquid flow (20) is, for example, constructed as a valve. The actions of the automated transport means (3), the docking/undocking of the split valve device, and the opening/closing of the split valve device are controlled by a control unit CU1(7).
**Figure 2** shows various embodiments of the automated transport means (3). a) the automated transport means (3) comprises a conveyer system, where individual storage vessels (1) are transported along rails into the docking position on top of the first reaction vessel RV1 (4) and back. b) the automated transport means (3) comprises a "carousel type" conveyer system, where individual storage vessels (1) are transported by rotation of a wheel into the docking position on top of the first reaction vessel RV1 (4) and back. c) the automated transport means (3) comprises a robotic arm, which engages with an individual storage vessel (1) via a gripping device (24), and shifts it into the docking position on top of the first reaction vessel RV1 (4) and back.
**Figure 3** shows one embodiment of the apparatus according to the present invention comprising one first reaction vessel RV1 (4) connected to a second reaction vessel RV2 (10) via a liquid connection line (6). A device controlling liquid flow (20) is integrated into the liquid connection line (6). The storage vessel (1) comprising a passive part (2) of a split valve device, the automated transport means (3) and the first reaction vessel RV1 (4) with the active part (5) of said split valve device are as described with respect to Figure 1. A cleaning device (17) allows to clean the active part (5) of the split valve device on the first reaction vessel RV1 (4). The first reaction vessel RV1 (4) and the second reaction vessel RV2 (10) are each equipped with a connection (21) to a source of protecting gas, with a pressure controller (23), and with a connection (22) to a vacuum source. Various liquid lines (11) allow for addition of solvent and liquid reagents. Liquid flow from the first reaction vessel (4) to the second reaction vessel RV2 (10) may be driven by any suitable means, e.g. by an overpressure applied to first reaction vessel RV1 (4), by gravity, by a vacuum applied to second reaction vessel RV2 (10), or by a pump [not pictured]. Liquid may be drained from the second reaction vessel RV2 (10) via a drain (12), in which a device controlling liquid flow (20) is integrated. A control unit CU1 (7) controls at least the actions of the actions of the automated transport means (3), the docking/undocking of the split valve device, the opening/closing of the split valve device, the devices controlling liquid flow (20), and the cleaning device (17). The control unit CU1 (7) may further control the dosing of solvent and reagents via the liquid lines (11), the settings of the pressure controller (23), the flow of protective gas into the reaction vessels RV1 and RV2, and/or the vacuum suction applied to the reaction vessels RV1 and RV2.
**Figure 4** shows one embodiment of the apparatus according to the present invention comprising three separate synthesis lines, each comprising one first reaction vessel RV1 (4) connected to a second reaction vessel RV2 (10). A single automated transport means (3) allows to bring a selected storage vessel (1) to a selected first reaction vessel RV1 for docking and material transfer. The other elements of the figure are as set out with respect to Figure 3. This embodiment of the apparatus thus allows to carry out three independent and different synthesis reactions in parallel.
**Figures 5** shows another embodiment of the apparatus according to the present invention. Each of the storage vessels (1) comprises a liquid inlet (8) feeding into a rinsing means (28) inside the storage vessel (1). A mobile solvent line (9) may be connected to the liquid inlet (8) of any storage vessel (1). Further, liquid lines (11) are each connected to a liquid inlet (8) feeding into a rinsing means inside the first reaction vessel RV1 (4) and inside the second reaction vessel RV2 (10), respectively. An additional liquid port (29) is provided on the first reaction vessel RV1 (4). This may allow for connection to incoming or outgoing lines. Both reaction vessels (4, 10) further comprise a mixing device (14), -e.g. a stirrer-, a sensor (16) -e.g. a temperature sensor, a pressure sensor, a level sensor, a turbidity sensor, an optical sensor, a conductivity sensor, an impedance sensor-, and a heating/cooling device (19); the second reaction vessel RV2 (10) additionally comprises a separation means (15) installed in the lower part of the vessel. This separation means (15) may allow to separate the carrier with the growing polymer chain from the remaining components of the reaction mixture by retaining the carrier inside the second reaction vessel RV2 (10) while draining the majority of other components via the drain (12). The cleaning device (17), which in this embodiment comprises a passive part (2) of the split valve device, allows to clean the active part (5) of the split valve device on the first reaction vessel RV1 (4). The liquid used for the cleaning process may be drained via a waste line (13). A monitoring device (18) -e.g. a scale- allows to verify whether material transfer from the storage vessel (1) to the first reaction vessel RV1 (4) is sufficiently complete. The other elements of the figure are as set out with respect to Figure 3.
**Figure 6** shows one embodiment of the apparatus according to the present invention comprising two first reaction vessels RV1 (4), each connected to two second reaction vessels RV2 (10). A single automated transport means (3) allows to bring a selected storage vessel (1) to a selected first reaction vessel RV1 (4) for docking and material transfer. The other elements of the figure are as set out with respect to Figure 3. This embodiment of the apparatus thus allows to carry out two independent and different synthesis reactions in parallel. In addition, two first reaction vessels RV1 (4) may be used in parallel for preparing building block solutions to be added to the same second reaction vessel RV2 (10).

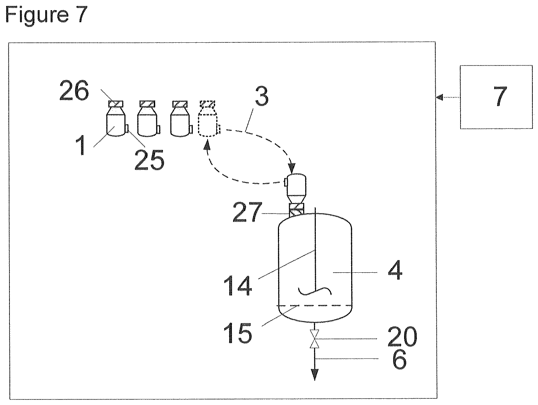

**Figure 7** shows another embodiment of the apparatus according to the present invention, which comprises a plurality of storage vessels (1), each comprising a first solid material transfer port (26) and a gripper plate (25). An automated transport means (3) selects a storage vessel (1), transfers said storage vessel (1) to a first reaction vessel RV1 (4), and aligns the first solid material transfer port (26) of the storage vessel with a second solid material transfer port (27) on the first reaction vessel RV1 (4). The solid material transfer ports (26, 27) are docked together and subsequently opened to allow for passage of solid material from the storage vessel (1) into the first reaction vessel RV1 (4). After the transfer, the solid material transfer ports (26, 27) are closed, the vessels undocked, and the storage vessel (1) is transported away from the first reaction vessel RV1 (4). Liquid may be drained from the first reaction vessel RV1 (4) via a liquid connection line (6). A device controlling liquid flow (20) is integrated into the liquid connection line (6). The actions of the automated transport means (3), the docking/undocking of the solid material transfer ports (26, 27), and the opening/closing of the solid material transfer ports (26, 27) are controlled by a control unit CU1 (7). In the embodiment pictured, the first reaction vessel RV1 (4) further comprises a mixing device (14) and a separation means (15) installed in the lower part of the vessel.

## Claims

1. An apparatus for performing iterative polymer synthesis, comprising:
a) a plurality of movable, closable storage vessels (1), which are shaped so as to enable transport by an automated means (3) and each comprise the passive part (2) of a split valve device, which is suitable for the transfer of solid material;
b) at least one first reaction vessel RV1 (4), which comprises the active part (5) of said split valve device;
c) an automated transport means (3) suitable for bringing a defined sequence of individual storage vessels (1) to a specific first reaction vessel RV1 (4) and away from it, wherein the automated transport means (3) is capable of aligning said passive part (2) of the split valve device with said active part (5) of the split valve device with sufficient precision so as to enable their docking to each other; and
d) at least one control unit CU1 (7) controlling the actions of the automated transport means (3) and of the split valve device (2, 5).

2. The apparatus according to claim 1, wherein the automated transport means comprises a robot or a conveyor device, preferably wherein the automated transport means comprises a robotic arm equipped with a gripping device (24).

3. The apparatus according to claims 1 or 2, wherein each of the storage vessels (1) comprises a liquid inlet (8), which allows connection to a mobile solvent line (9).

4. The apparatus according to any one of claims 1 to 3, wherein the first reaction vessel RV1 (4) comprises a liquid inlet (8), which allows connection to a liquid line (11).

5. The apparatus according to any one of claims 1 to 4, further comprising a device M (18) for monitoring the extent of material transfer from the storage vessel to the first reaction vessel RV1 (4).

6. The apparatus according to any one of claims 1 to 5, further comprising:
e) at least one second reaction vessel RV2 (10) connected to at least one of the one or more first reaction vessel(s) RV1 (4)
f) a device (20) for controlling liquid flow from said first reaction vessel(s) RV1 (4) into said connected second reaction vessel(s) RV2 (10), which is controlled by the control unit CU1 (7).

7. The apparatus according to any one of claims 1 to 6, further comprising at least one cleaning device (17) for the active part (5) of the split valve device of the first reaction vessel(s) RV1 (4), wherein the actions of the cleaning device are controlled by the control unit CU1 (7).

8. The apparatus according to claims 6 or 7, further comprising a waste line (13) allowing to drain liquid from the first reaction vessel RV1 (4) and from a liquid connection line (6) connecting the first reaction vessel RV1 (4) with the second reaction vessel RV2 (10), without passage of the liquid through the second reaction vessel RV2 (10).

9. The apparatus according to any one of claims 6 to 8, comprising a number of n first reaction vessels RV1 (4) and a number of m second reaction vessels RV2 (10), wherein n and m are integers chosen independently from the range of 1 to 10.

10. An apparatus according to any one of claims 1 to 9 comprising at least one reaction vessel RV1 (4) or RV2 (10), wherein said reaction vessel or reaction vessels further comprise(s) one or more elements selected independently for each reaction vessel from the group consisting of a sensor (16), a heating and/or cooling device (19), a mixing device (14), a liquid line (11), a liquid port (29), a means (28) for rinsing the inner walls of the reaction vessel, and a means (15) for separating the carrier with the growing polymer chain from the remaining components of the reaction mixture.

11. An apparatus suitable for performing iterative polymer synthesis, comprising:
a) a plurality of movable, closable storage vessels (1), which are shaped so as to enable transport by an automated means (3) and each comprise a first transfer port (2) suitable for the transfer of solid material;
b) at least one first reaction vessel RV1 (4), which comprises a second transfer port (5) suitable for the transfer of solid material, wherein:
• said first transfer port (26) of the first reaction vessel RV1 (4) is constructed and arranged such that it can be releasably docked to said second transfer port (27) of each storage vessel, thereby forming a connection between both vessels; and
• material can pass though said transfer ports (26, 27) when in connected state, but not in disconnected state;
c) an automated transport means (3) suitable for bringing a defined sequence of individual storage vessels (1) to a specific first reaction vessel RV1 (4) and away from it, wherein the automated transport means is capable of aligning said transfer port (2) on the storage vessel (1) with said transfer port (5) on the first reaction vessel RV1 (4) with sufficient precision to enable their docking to each other; and
d) at least one control unit CU1 (7) controlling the actions of said automated transport means (3), the docking of said ports (2, 5), and the opening and closing of the port.

12. A movable, closable storage vessel (1) for use in the apparatus according to any one of claims 1 to 10, which is shaped so as to enable transport by an automated transport means and which comprises a machine-readable tag, a liquid inlet (8) connectable to a mobile solvent line (14), and the passive part (2) of a split valve device, which is suitable for the transfer of solid material.

13. A method of automated iterative polymer synthesis, comprising the use of an apparatus according to any one of claims 1 to 0, or of a storage vessel (1) according to claim 12.

14. A method of iterative polymer synthesis, wherein the following steps 1) to 10) are carried out at least once in automated fashion:
1) Providing a plurality of movable, closable storage vessels (1), which are shaped so as to enable transport by an automated means (3) and each comprise a passive part (2) of a split valve device suitable for the transfer of solid material, wherein each of the storage vessels (1) contains a defined amount of a building block B in solid form to be used in one cycle of the iterative polymer synthesis process;
2) Transferring a specific storage vessel (1) selected from said plurality of storage vessels (1) to a first reaction vessel RV1 (4), which comprises an active part (5) of said split valve device using an automated transport means (3);
3) Aligning and docking the passive part (2) of the split valve device on said storage vessel (1) to the active part (5) of the split valve device on said first reaction vessel RV1 (4);
4) Opening the split valve device (2, 5) and transferring the building block B from said storage vessel into said first reaction vessel RV1 (4);
5) Dissolving said building block B by addition of a suitable solvent, thereby forming a solution of the building block B inside the first reaction vessel RV1 (4);
6) Transfer of the solution obtained in step 5), to a second reaction vessel RV2 (10) containing a carrier, to which a molecule C is tethered, thereby obtaining a reaction mixture containing the building block B and the carrier with the molecule C;
7) Cleaning the first reaction vessel RV1 (4), including the active part (5) of the split valve device, by rinsing with solvent, which may be the same as or different from the solvent used in step 5);
8) Incubating of the reaction mixture obtained in step 6) under conditions, which allow for the formation of a chemical bond between the building block B and the molecule C so as to form a molecule C', which is extended by one building block unit;
9) Retaining the carrier with the extended molecule C' inside the second reaction vessel RV2 (10), while purging a liquid comprising byproducts and residual educts of the coupling reaction from the second reaction vessel RV2 (10);
10) Conditioning the carrier with the extended molecule C' for the next coupling round with the extended molecule C' as molecule C.

15. A method of iterative polymer synthesis, wherein the following steps i) to x) are carried out at least once in automated fashion:
i) Providing a plurality of movable, closable storage vessels (1), which are shaped so as to enable transport by an automated means (3) and each comprise a passive part (2) of a split valve device suitable for the transfer of solid material, wherein each of the storage vessels (1) contains a defined amount of a building block B in solid form to be used in one cycle of the iterative polymer synthesis process;
ii) Providing a first reaction vessel RV1 (4), which comprises an active part (5) of said split valve device, and which contains a carrier, to which a molecule C is tethered;
iii) Transferring a specific storage vessel (1) selected from said plurality of storage vessels (1) to said first reaction vessel RV1 (4) containing the carrier with the molecule C using an automated transport means (3);
iv) Aligning and docking of the passive part (2) of the split valve device on said storage vessel (1) to the active part (5) of the split valve device on said first reaction vessel RV1 (4);
v) Opening of the split valve device and transferring the amount of the building block B from said storage vessel into said first reaction vessel RV1 (4);
vi) Dissolving said building block B by addition of a suitable solvent, thereby forming a reaction mixture containing the building block B and the carrier with the molecule C inside the first reaction vessel RV1 (4);
vii)Incubation of the reaction mixture obtained in step vi) under conditions, which allow for the formation of a chemical bond between the building block B and the molecule C so as to form a molecule C', which is extended by one building block unit;
viii) Retaining the carrier with the extended molecule C' inside the first reaction vessel RV1 (4), while purging a liquid comprising byproducts and residual educts of the coupling reaction from the first reaction vessel RV1 (4)
ix) Rinsing the first reaction vessel RV1 (4), including the active part (5) of the split valve device, with a solvent, which may be the same as or different from the solvent used in step vi);
x) Conditioning the carrier with the extended molecule C' for the next coupling round with the extended molecule C' as molecule C.
